(19) Europäisches Patentamt
European Patent Office
Office européen des brevets

(11) Publication number: 0 172 035 B1

# (12) EUROPEAN PATENT SPECIFICATION

(45) Date of publication of patent specification: 09.10.91

(51) Int. Cl.⁵: A61F 13/46

(21) Application number: 85305844.4

(22) Date of filing: 16.08.85

## (54) Thin soft absorbent product.

(30) Priority: 17.08.84 US 641545
17.08.84 US 641549

(43) Date of publication of application:
19.02.86 Bulletin 86/08

(45) Publication of the grant of the patent:
09.10.91 Bulletin 91/41

(84) Designated Contracting States:
AT BE CH DE FR GB IT LI LU NL SE

(56) References cited:
EP-A- 0 108 637

(73) Proprietor: McNEIL-PPC, INC.
One Johnson & Johnson Plaza
New Brunswick, New Jersey 08933(US)

(72) Inventor: Iskra, Michael J.
Rd 6, 5 Greenhills Drive
Flemington New Jersey(US)

(74) Representative: Jones, Alan John et al
CARPMAELS & RANSFORD 43 Bloomsbury Square
London, WC1A 2RA(GB)

EP 0 172 035 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to new and improved thin, soft, absorbent products, and more particularly, to new and improved soft compressed composites incorporating superabsorbent material and which absorb large quantities of liquids.

Disposable absorbent products have been known for some time, including such products as disposable diapers, sanitary napkins, wound dressings, bandages, incontinence pads, and the like. These products incorporate an absorbent batt which is used to absorb and hold or contain body fluids. Initially, in many of these products, especially diapers and sanitary napkins, the absorbent batt comprised what is termed "wadding" or plies of tissues. The wadding was disposed between an impermeable backing and a permeable facing and the plies of tissue were used to absorb and, hopefully, contain the liquid within the product. A diaper which utilizes such an absorbent batt is disclosed in U.S. Reissue Patent No. 26,151.

The wadding type of batt was replaced, for the most part, by an improved absorbent batt which comprises what is termed "fluffed wood pulp fibers". This absorbent batt comprises a layer of individualised wood pulp fibers with the layer having substantial thickness. A diaper which incorporates such a fluffed wood pulp absorbent batt is described in U.S. Patent No. 2,788,003. This diaper had improved absorbent capacity and somewhat better containment than a diaper using a wadding layer. Also the fluffed wood pulp layer is quite soft, flexible and conformable and hence, produces an improved diaper over diapers using wadding as the absorbent layer.

Though the fluffed wood pulp absorbent batts have improved capacity, the efficiency with which the capacity is used in a diaper or sanitary napkin is poor. The reason for this is that the fluid to be absorbed is generally deposited in a localized area within the absorbent batt and the ability for the fluid to move along the plane of the batt is poor. The fluid follows the path of least resistance and consequently moves to the closest edge of the batt where it generally is no longer contained and the product leaks. Furthermore, the wood pulp batts lack stability, e.g. when a diaper is being worn, the batt tends to break up creating bunching.

U.S. Patent No. 3,017,304 discloses an absorbent product which incorporates in the product a densified, paper-like layer. This paper-like layer acts as a wick, i.e. liquid which is placed on the layer tends to move rapidly along the plane of the layer. When incorporated in combination with fluffed wood pulp fiber, the resultant product uses the absorbent capacity of the fluffed wood pulp much more efficiently. Diapers which incorporate this paper-like layer combined with fluffed wood pulp are disclosed and described in U.S. Patent Nos. 3,612,055 and 3,938,522. This concept of combining a wicking layer or capillary skin with fluffed wood pulp fibers has gained wide acceptance in many absorbent products including disposable diapers and sanitary napkins. Even though these products make much greater use of the capacity of the absorbent batt, they still do not totally contain the absorbed liquid. It is probable that these products will leak before the full capacity of the batt is used for absorption. This is especially true if pressure is placed on the batt while wet, for example a baby sitting down on a previously wetted diaper will often cause the batt to leak. Although the batt is somewhat stabilized by the paper-like densified skin, it may crack and separate.

Recently, elastic leg diapers or stretch diapers have been introduced into the market place. Though these diapers provide no better absorbent batt than flat diapers or the prior art diapers, they have indicated improved containment of liquid. Such diapers are disclosed and described in U.S. Patent Nos. 3,860,003; 4,050,462 and 4,324,245. Though the containment features are better than the prior art products, the elasticized products fit more tightly, permitting less air circulation. Frequently, this can become irritating to the skin and the tighter the elastic or the more close fitting the diaper, the greater the irritation. This is especially true adjacent the area where the elastic leg portion of the product contacts the wearer.

A number of years ago "superabsorbent" materials, i.e. materials which will absorb many times their weight of liquid, were developed. Since the development of such materials, people have been trying to incorporate them in absorbent products such as diapers and sanitary napkins to enhance the absorptive performance of these products. Theoretically, a minimum amount of superabsorbent incorporated in a product would make that product perform as well or better than the prior art products. Perhaps one of the first products to incorporate such a superabsorbent material in a disposable diaper is disclosed in U.S. Patent No. 3,670,731. This patent discloses an absorbent dressing comprising an absorbent layer sandwiched between a liquid-permeable facing and a liquid-impermeable backing sheet. The absorbent layer contains water insoluble cross-linked hydrocolloid polymer as the superabsorbent material.

Even though superabsorbent materials have been available for some time, they have not gained wide acceptance in absorbent products such as disposable diapers and sanitary napkins. A primary reason for this lack of acceptance of the superabsorbents is failure to develop a product capable of economically utilizing the highly increased absorptive capacity of the superabsorbent material. In order to economically

utilize a superabsorbent, the liquid being absorbed must be transported to the superabsorbent material. In other words, the superabsorbent material must be placed in contact with the liquid. Furthermore, as the superabsorbent material absorbs the liquid, it must be allowed to swell. If the superabsorbent is prevented from swelling, it will cease absorbing liquid. Hence, if the superabsorbent material is to function in diapers and sanitary napkins wherein the liquid to be absorbed is placed in a small void area, the structure of the absorbent layer containing superabsorbent materials appears to be critical. Over the years a number of techniques have been disclosed in an attempt to provide structures which make efficient use of the superabsorbent material. Such products are disclosed in U.S. Patent Nos. 4,103,062,; 4,102,340 and 4,235,237. In addition, methods for incorporating superabsorbents into suitable layers or suitable configurations which can be placed in an absorbent product are disclosed in U.S. Patent Nos. 4,186,165; 4,340,057 and 4,364,992. To date, none of these products has met with any substantial commercial success.

In copending Application EP-A-0 108 637, a particularly useful compressed composite is formed. This application is hereby incorporated by reference. The compressed composite product is preferably made from a nonwoven fabric such as polyester. The fabric has associated with it at least 200 percent by weight of superabsorbent to form an absorbing layer. In order to provide a product which will not only absorb liquid but also transport liquid, wood pulp fibers or other suitable wicking materials are cast in a layer on at least one side of the absorbing layer. The product is then compressed to yield a very high liquid absorbing product. However, the resulting compressed composite is quite stiff, and hence requires softening to provide flexibility for utilization in products such as diapers and the like. The flexibility provided needs to be permanent, i.e. the surrounding environment, handling of the product, and its subsequent use will not affect the softness and flexibility.

The present invention provides a new and improved absorbent composite structure which utilizes a substantial portion of the absorptive capacity of superabsorbent materials and yet is soft and flexible. This soft, flexible composite makes use of the capacity of the superabsorbent materials. Furthermore, the composite retains its substantially completely stable state though rendered soft and flexible. Whether wet or dry, the composite does not break, bunch or separate. The soft composite retains absorbed liquid without yielding any of the liquid when the composite is under pressure.

The present invention provides an absorbent structure comprising a fibrous web of resilient fibers, said web having a basis weight less than 135.6 $g/m^2$ ($4oz/yd^2$), an initial dry bulk of at least 20 $cm^3/g$, a dry bulk recovery of at least 30 percent and a wet bulk of at least 30 $cm^3/g$, and superabsorbent disposed in amongst the fibers of said web in an amount of at least 200 percent by weight based on said web weight, said structure having a Taber stiffness value of less than 50 and being microcorrugated.

Preferably, the structure has a Taber stiffness value of less than 25, most preferably less than 10.

Advantageously, the structure further comprise a wicking layer, for instance of wood pulp fibers, placed on one or preferably both faces of said fibrous web.

The absorbent structure of the present invention may be used as the absorbing layer of the compressed composite product discussed above.

The present invention also provides disposable absorbent products, such as sanitary napkins and diapers including such an absorbent structure.

The fibrous web used as a basis for the absorbing layer is preferably a low density resilient fibrous web consisting of randomly disposed fibers which result in a web having a dry bulk recovery of at least 30 percent and initial dry bulk of at least 20 $cm^3/g$, a wet bulk of at least 30 $cm^3/g$, and a weight less than 135.6 $g/m^2$ (4 $oz/yd^2$), preferably less than 101.7 $g/m^2$ (3 $oz/yd^2$). The fibrous web is used to spacially distribute superabsorbent material so that upon exposure to an aqueous fluid, swelling occurs with minimal interference from adjacent superabsorbent material.

After formation of the absorbing layer, the wicking layer may, if desired, be formed as a superposed layer on the absorbing layer. The wicking layer generally is a layer of wood pulp fibers in an amount of 135.6 $g/m^2$ (4 $oz/yd^2$) or more. The two layers, while still in a moist state of at least about 10 percent moisture are compressed to form a compressed composite product.

The absorbent structure of the invention, with or without a wicking layer, is too stiff for most uses due to the high loading of superabsorbent in the absorbing layer. It has been discovered that such an absorbent structure can be softened to a Taber stiffness of 50 or less by microcorrugation.

The present invention also provides a method for preparing a soft, flexible absorbent structure comprising the steps of:

a) preparing a fibrous web of resilient fibers, said web having a basis weight less than 135.6 $g/m^2$ (4 $oz/yd^2$), an initial dry bulk of at least 20 $cm^3/g$, a dry bulk recovery of at least 30 percent and a wet bulk of at least 30 $cm^3/g$, said web having superabsorbent disposed in amongst its fibers in an amount of at least 200 percent by weight based on said web weight;

b) drying said web containing superabsorbent to a moisture content less than 25 percent; and

c) microcorrugating said web to provide a Taber stiffness of less than 50.

Preferably, the moisture content is reduced to less than 10% prior to microcorrugating.

The microcorrugating process preferably consists of passing the web through fluted intermeshing rolls having sufficient pressure to fracture the superabsorbent and form cross-directional hinge lines. Alternatively or additionally the web can be passed through an embossing roll with rings set to fracture the superabsorbent and place lines in the product in a machine direction, thus providing hinge lines in the machine direction.

Advantageously, either before or after said microcorrugating step (c) a wicking layer is placed on one or preferably both faces of said fibrous web and said structure including said wicking layer or layers is subjected to perf-embossing following the microcorrugating step.

The perf-embossing is carried out by known techniques such as that exemplified in U.S. Patent 3,817,827.

In order for the absorbent structure to be softened and reduced substantially in Taber stiffness it is necessary to attain the glass transition temperature of the superabsorbent material so that the superabsorbent polymer is brittle and can be reduced in size effectively by the mechanical working and crushing provided by the microcorrugating and optionally the perf-embossing. The glass transition temperature is reached by reducing the moisture content sufficiently to permit satisfactory operation at the temperature of the room in which the operation is being carried out. For most superabsorbent materials the satisfactory moisture content is less than 25%, preferably less than 10%, by weight of moisture of the absorbent composite structure.

In the microcorrugating process step, a Taber stiffness is reduced by at least 50% and frequently by at least 70%. During any subsequent perf-embossing step, the Taber stiffness is reduced to a value generally less than 15 but in each instance to a value less than 25.

The perf-embossing step, when used, should provide sufficient impact points on the product to further reduce the superabsorbent polymer particle size. Generally, if the impact points are no more than 6.35 mm (1/4 inch) apart and are somewhat continuous, a satisfactory change in Taber stiffness will be achieved.

In the process of perf-embossing, the composite is passed through a pair of rolls which have knuckles and which intermesh to shear the composite structure in the desired fashion. When looking at the structure after it has been perf-embossed, there are raised areas produced by lower knuckles and depressions produced by upper knuckles. If the structure is viewed from the other side, the raised areas become depressions and the depressions become raised areas. The depressions are densified regions which hold and wick liquid. Interconnecting the raised areas and the depressions are intermediate portions which have received most of the mechanical working which reduces the superabsorbent polymer size and fuses the layers of the composite together in the shear areas.

At locations where the upper knuckles pass very close the lower knuckles of the rolls, the work applied to the structure exceeds the strength of the structure and produces apertures in it. The length of the apertures can be varied by controlling the overlap of the upper knuckles and lower knuckles or the size of the knuckles of the rolls. Though the flexibility of the structure is increased by the apertures, the overall strength of the product may be decreased. Therefore, the preferred product of the present invention employs controlled portions of both apertures and partially fractured or sheared regions.

The fibrous web which contains the superabsorbent and forms the basic absorbing layer for the absorbent composite of the present invention is of substantially high loft and upon dry compression followed by a release has a tendency to return substantially to its original thickness. For instance, fibrous webs formed from synthetic staple fibers, such as polyethylene, polypropylene, polyester, nylon or bicomponent fibers are particularly desirable. Melt blown fibrous webs also are suitable. Generally, the fibers are air-laid or melt blown to form a web which, if needed, is then stabilized. Stabilization may be achieved by heat-through bonding, adhesive bonding or point embossing with heat or adhesive. The stabilizing process is selected according to the fibers used and the process used to form the web. Suitable procedures for forming a web include carding, wet-laying, air-laying, or combinations of these, melt blowing and other suitable known techniques. The fibrous web has a dry bulk recovery of at least 30 percent and an initial dry bulk of at least 20 $cm^3/gm$ and a wet bulk of at least 30 $cm^3/gm$. The fibrous web generally has a weight less than 135.6 $g/m^2$ (4 $oz/yd^2$), preferably less than 101.7 $g/m^2$ (3 $oz/yd^2$).

A wicking layer, generally of wood pulp fibers, may, if desired, be placed on at least one side of the superabsorbent containing fibrous web and in the presence of about 10 percent moisture or more, the product is compressed. The resulting compressed composite generally possesses a Taber stiffness in the machine direction of at least 130 and sometimes as high as 350. If the structure is to be perf-embossed, it has been found to be necessary to include such a wicking layer.

In one embodiment, a blend of staple polyester fibers with a minor portion of fusible fibers are air-laid to form a web. The web is subsequently lightly bonded by passing hot air through the fibers making the fusible fibers tacky so as to stick to each other and the staple fibers to provide some degree of integrity to the web structure.

The superabsorbent material present in an intermittently dispersed form in the absorbing layer is generally a water-insoluble but water-swellable polymeric substance capable of absorbing water in an amount which is at least 10 times the weight of the substance in its dry form. The superabsorbent material is in the form of particles which may be in the shape of fibers, spheres, bits of film, globules of the like, or may be applied in the form of a liquid monomer solution which is subsequently polymerized. Generally, the polymerized monomer solution provides globules and bits of film-like particles in the structure.

In one type of superabsorbent material, the particles or fibers may be described chemically as having a backbone of natural synthetic polymers with hydrophilic groups or polymers containing hydrophilic groups being chemically bonded to the backbone or in intimate admixture therewith. Included in this class of materials are such modified natural and regenerated polymers as polysaccharides including, for example, cellulose and starch and regenerated cellulose which are modified by being carboxyalkylated, phosphonoalkylated, sulphoalkylated or phosphorylated to render them highly hydrophilic. Such modified polymers may also be cross-linked to improve their water-insolubility.

These same polysaccharides may also serve, for example, as the backbone onto which other polymer moieties may be bonded by graft copolymerization techniques. Such grafted polysaccharides and their method of manufacture are described in U.S. Patent No. 4,105,033 to Chatterjee et al. and may be described as polysaccharide chains having grafted thereon a hydrophilic chain of the general formula

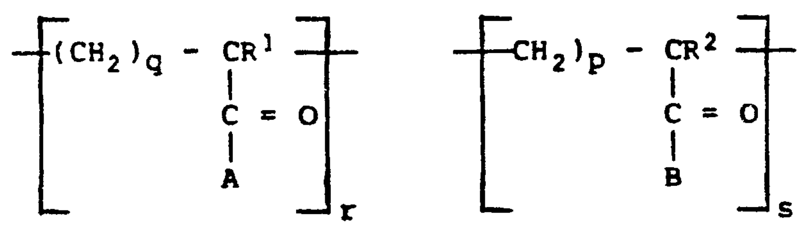

wherein A and B are selected from the group consisting of $-OR^3$, -O (alkali metal), $-OHNH_3$, $-NH_2$, wherein $R^1$, $R^2$ and $R^3$ are selected from the group consisting of hydrogen and alkyl having 1 to 4 or more carbon atoms, wherein r is an integer having a value of 0 to about 5000 or more, s is an integer having a value of 0 to about 5000 or more, r plus s is at least 500, p is an integer having a value of zero or 1 and q is an integer having a value of 1 to 4. The preferred hydrophilic chains are hydrolyzed polyacrylonitrile chains and copolymers of polyacrylamide and polysodium acrylate.

In addition to modified natural and regenerated polymers, the hydrocolloid particle component may comprise wholly synthetic hydrophilic particles. Examples of those now known in the art are polyacrylonitrile fibers which may be modified by grafting moieties thereon such as polyvinyl alcohol chains, polyvinyl alcohol itself, hydrophilic polyurethane, poly(alkyl phosphonates), partially hydrolyzed polyacrylamides (e.g. poly(N-N-dimethyl acrylamide), sulfonated polystyrene, or a class of poly-(alkylene oxide). These highly hydrophilic synthetic polymers may be modified by other chemical treatments such as cross-linking or hydrolysis. Further examples known in the art are the non-ionic hydrophilic polymers such as polyoxyethylene, polyoxypropylene and mixtures thereof which have been suitably cross-linked, either chemically or by irradiation. Still another more recent type is a derivative of isobutylene-maleic anhydride copolymer.

Hydrophilic polymers formed from water-soluble acrylate monomers, such as sodium, potassium, ammonium (or combination of cations), acrylate, may be placed on the absorbing layer by spraying or otherwise placing a solution thereon followed by polymerization and cross-linking, for example, by irradiation.

In addition, naturally occurring materials such as gums, may be used. For instance, guar gum is suitable.

The superabsorbent material is combined with the fibrous web by any means suitable to distribute the superabsorbent material therein while trying to minimize interference by one superabsorbent entity with another upon the swelling of the first. If the superabsorbent material is a powder it may be sprinkled onto the fibrous web either in dry form or the web may be moistened. If the superabsorbent is in granular form it may be desirable to slightly moisten the superabsorbent before placing it in contact with the web. The superabsorbent material will contain particles which range in size from about 0.005mm in diameter to

globules that are continuous along fibers for a distance up to several centimeters (inches).

Another method of placing the superabsorbent in the web is spraying a monomer solution on the web or saturating the web with a monomer solution followed by polymerization of the monomer. One typical way to polymerize the monomer is by use of irradiation. It is desirable to place the superabsorbent somewhat evenly throughout the fibrous web. However, even if the superabsorbent is powderlike and in the form of a layer, it tends to function better than such a layer has in previously known products. It may be desirable to place more superabsorbent in one area than in another and/or to place the superabsorbent in the structure in predetermined patterns.

Any superabsorbent which absorbs large amounts of liquids is suitable for use in the absorbing layer of the present invention.

The fibrous web containing the superabsorbent, whether or not it has any wicking layers thereon, tends to be stiff and substantially non-flexible. Since the end uses of the fibrous web require that the web be soft, flexible and pliable, it has been discovered that microcorrugation of the fibrous web provides the necessary reduction in stiffness without damaging the properties of the fibrous web which are desirable for its end use. Frequently, the Taber stiffness of a web containing at least 200 percent superabsorbent exceeds 300 Taber stiffness (g/linear cm) in the machine direction. In the cross-direction, the Taber stiffness generally exceeds 70. In order to have a product satisfactory for use in disposable products, such as diapers and sanitary napkins, it is necessary to reduce the Taber stiffness value to less than 50, preferably less than 25. The Taber stiffness value is obtained in accordance with the procedure found at ASTM D 2969. All Taber stiffness values are expressed in g/linear cm.

The absorbing layer comprised of the fibrous web and superabsorbent may be treated by microcorrugating to reduce the Taber stiffness on the layer alone or after the layer has been combined with a wicking layer.

The wicking layer, when present, is comprised of hydrophilic fibers, such as rayon fibers, cellulosic fibers, peat moss, acrylic fibers, or mixtures thereof. The cellulosic fibers include wood pulp fibers, cotton linters, and the like. The wood pulp fibers generally are those that are used to form the fluff or fibrous batt layer in conventional absorbent products such as disposable diapers, sanitary napkins, etc. Other cellulosic fibers that might be used are rayon fibers, flax, hemp, jute, ramie, cotton and the like. The fibers or peat moss or mixtures thereof are placed in such a way as to form a layer in which the particles are close to one another so as to provide a higher capillary pressure to promote wicking of liquid in the plane of the layer.

What appears to be only a small difference in capillary pressure is all that is required for one layer to attract and drain liquid from an adjacent layer. The force causing a liquid to enter a cylindrical capillary is expressed by the equation:-

$$P = \frac{(2\nu \cos \theta)}{r}$$

wherein the force is represented by the capillary pressure and

- P is the capillary pressure,
- is the surface tension of the liquid,
- 0 is the liquid-fiber contact angle and
- r is the capillary radius.

With a given liquid, the pressure (capillary force) increases with the cosine of the liquid-fiber contact angle (reaching a maximum where the angle is zero) and also increases with narrower capillary radii so that narrower capillaries will draw liquid from wider ones.

The relative wickability between a first fibrous layer and a second layer is affected by both the relative densities of the layers and the relative wettability of the individual fibers in each layer. The individual fibers of the second layer preferably have substantially smaller liquid fiber contact angles than those of the first fibrous layer overcoming the density difference and providing a significant overall increase in capillary pressure to absorb liquid into the second layer.

The fibers of the second layer of fibers (or particles) and/or the density of the layer are selected to create a significant difference in capillary pressure from the first fibrous layer.

The second fibrous (or particle) layer is generally comprised of fibers having a lower liquid-contact angle or wherein the layer is provided with a narrower capillary radii. Examples of such fibers include hydrophilic fibers such as rayon fibers, cellulosic fibers, or peat moss, or mixtures thereof, or acrylic fibers,

or the like. Cellulosic fibers include wood pulp fibers, cotton linters and the like.

The wicking layer can be preformed and placed next to the absorbing layer before compression or the wicking layer particles can be air-laid, mechanically entangled therewith, or wet-laid on to the absorbing layer before compression.

The transition zone is a region formed at the junction of the absorbing layer and the wicking layer. Some of the particles, e.g. fibers, of the wicking layer extend into and become integral with the absorbing layer. The region in which the majority of the extending particles lie is identified as the transition zone. In the transition zone, there is a composite of absorbing layer fibers, superabsorbent material, and wicking layer particles. The wicking layer particles which have extended into the absorbing layer are in intimate contact with some of the superabsorbent material of the absorbing layer. This permits the liquid to commence its migration in the z direction to reach the superabsorbent material. As the liquid progresses in the z direction, the superabsorbent material becomes soft and releases the absorbing layer fibers which permit the absorbing layer to return substantially to its uncompressed thickness or more. As the absorbing layer returns to its uncompressed thickness, larger void areas are provided for storage of the liquid and for increased swelling of the superabsorbent material as it absorbs the liquid residing in the void areas. The absorbing layer tends to return to its uncompressed thickness or more, probably because of both the resiliency of the fibers and the swelling of the superabsorbent material.

In order for the absorbing layer fibrous web to provide the necessary medium for absorbing liquid, it is preferred that the fibrous web has an initial dry bulk of at least 20 cc/g,. a dry bulk recovery of at least 30 percent, (preferably 50 percent), a wet bulk of at least 30 cc/g, and a weight of less than 135.6 g/m$^2$ (4 oz/yd$^2$). The initial dry bulk is the area times thickness of the layer under a load of 68.95 Pa (0.01 pounds per square inch) calculated in cubic centimeters. This value is divided by the weight in grams in order to provide the measurement in cubic centimeters per gram. The dry bulk recovery is obtained by subjecting the web to a load of 12 kPa (1.75 psi) for five minutes, removing the load and allowing the web to rest for one minute, subjecting the web to a load of 68.95 Pa (0.01 psi) for one minute and then measuring the final dry bulk while under the 68.95 Pa (0.01 psi) load. The dry bulk recovery is the final bulk divided by the initial bulk expressed in percent. The wet bulk is measured in the same manner as the initial dry bulk except that the web has been saturated with water. It has been found that if the fibrous web is provided with a dry bulk recovery of at least 20 percent (preferably 50%), an initial dry bulk of at least 20 cc/g, a wet bulk of at least 30 cc/g, with a web weight of less than 135.6 g/m$^2$ (4 oz/yd$^2$), the fibrous web can retain superabsorbent material up to at least 1,500 percent of the dry basis weight of the web. It is preferable that the web contain 200 percent to 1,500 percent by weight, dry basis, superabsorbent to the dry basis weight of the web and most preferred is a range from 400 percent to 1,200 percent.

The means by which the absorbent structure is rendered flexible, pliable, and soft according to the present invention is by utilization of mechanical working. The mechanical working is identified as microcorrugating, followed, if desired, by perf-embossing.

The technique of microcorrugating is disclosed in U.S. Patent 4,116,892 to Schwarz. Although the technique in this patent is identified as one for stretching fibers in order to orient the fibers, the present invention does not wish to stretch but to mechanically work. Schwarz uses his technique to molecularly orient the fibers in a post molten state. The fibers used in the present invention are already oriented. Thus, additional stretching or further orientation might damage the fibers in the present invention.

It has been discovered that microcorrugating the absorbent structure defined in the present invention puts in hinge lines resulting in a flexible, pliable, soft feel. The rolls described in U.S. Patent 4,116,892 are set with a gap of 0.63 to 7.62 mm (0.025 - 0.30 inch) in order to break up the superabsorbent particles to a somewhat uniform size and create the desirable hinge lines described heretofore. If the absorbent structure of the present invention is simply put through rolls as to crush the structure, the product actually becomes stiffer and has a higher Taber stiffness value. The stretch to the fibers of the absorbent structure is less than 10 percent when the microcorrugating is performed.

It is easier to pass the absorbent structure through the microcorrugating rolls in the machine direction. However, it is acceptable to microcorrugate in the machine direction, thereby placing hinge lines in the product in the machine direction. This can be accomplished through rolls with embossing rings which are so placed as to provide the necessary hinge lines.

It has nonetheless been found desirable to pass the materials through the microcorrugating rolls in the machine direction and then to pass the material through rolls with embossing rings in order to microcorrugate in the cross direction.

It is highly desirable to reduce the moisture content of the absorbent structure to less than 10 percent before subjecting it to microcorrugating.

In addition to the tenderizing, softening and improving flexibility of the product, it has been noted that

the product absorbs liquid more rapidly than prior to the mechanical working treatment by microcorrugation. The quick absorption of liquid is particularly beneficial in a disposable diaper product.

It has been found that when a microcorrugated absorbent structure having at least one wicking layer is subjected to perf-embossing after it has been microcorrugated, the absorbent product is a flexible, pliable, soft product which retains substantially its original machine direction strength while having been mechanically worked in such a way as to improve further the absorption and reduce the Taber stiffness by a considerable amount, placing the final Taber stiffness below 25, and preferably below 15. The perf-embossing is carried out by known techniques such as that exemplified in U.S. Patent 3,817,827.

In order for the composite structure to be best treated, it is preferable to attain the glass transition temperature of the superabsorbent material so that the superabsorbent polymer is brittle and can be reduced in size effectively by the mechanical working and shearing provided by the perf-embossing. The glass transition temperature is reached by reducing the moisture content sufficiently to permit satisfactory operation at the temperature of the room in which the operation is being carried out. For most superabsorbent materials, the most satisfactory moisture content is less than 10% by weight of moisture of the composite structure.

In addition to the further tenderizing, softening and improving flexibility of the absorbent composite structure, it has been noticed that the product absorbs liquid in larger quantities than prior to the mechanical working procedure by microcorrugating and perf-embossing. Furthermore, the quick absorption of liquid by the product is not substantially decreased. These qualities are particularly beneficial for a compressed composite product used in a disposable diaper.

The present invention will now be described by way of example only, with reference to the accompanying drawings, in which:-

Figure 1 is a perspective view illustrating one type of starting material for the present invention;

Figure 2 is a perspective view of one embodiment of the present invention, wherein the starting material of Figure 1 has been microcorrugated;

Figure 3 is a perspective view of another type of starting material for use in the present invention;

Figure 4 is an enlarged cross-sectional view along lines 4-4 of Figure 3;

Figure 4A is an enlarged cross-sectional view along lines 4-4 of Figure 3 after compression;

Figure 5 is a perspective view illustrating yet another type of starting material for the present invention;

Figure 6 is an enlarged cross-sectional view of Figure 5 taken along line 6-6 of Figure 5;

Figure 6A is a cross-sectional view as in Figure 6 after compression of the product;

Figure 7 is a perspective view of another embodiment of the present invention;

Figure 8 is a perspective view of a disposable diaper including an embodiment of the present invention;

Figure 9 is a perspective view of a sanitary napkin including an embodiment of the present invention;

Figure 10 is an enlarged side elevational view of a portion of the embodiment shown in Figure 7;

Figure 11 is an enlarged side elevation view of a portion of the microcorrugating rolls;

Figure 12 is a graph depicting the test results of Example 2;

Figure 13 is a perspective view of the starting material of Figure 5 after the perf-embossing step;

Figure 14 is a perspective view of another diaper including a structure of the present invention with a portion broken away for clarity;

Figure 15 is a perspective view of another sanitary napkin including a structure of the present invention with a portion broken away for clarity;

Figure 16 is a graph depicting the test results of Example 3.

Referring now to the drawings, Figure 1 represents a perspective view of a starting material utilized to make an absorbent product of the present invention. The starting material 10 is a fibrous web containing at least 200 percent superabsorbent by weight of the web. The superabsorbent particles 14 are distributed substantially throughout the web 12.

Figure 2 denotes a fibrous web 20 which contains superabsorbent material 24. The fibrous web 20 has been microcorrugated to provide microcorrugations 22 in the fibrous web resulting in a softness and flexibility imparted to an otherwise stiff material.

Figure 3 represents a perspective view of another starting material for the absorbent structure of the present invention. The starting material 30 has a fibrous web as an absorbing layer 32. Interspersed and fixed in the absorbing layer, is superabsorbent material 36. Immediately associated with the absorbing layer is a wicking layer 34. Some portions of the wicking layer 34 extend into and become integral with the absorbing layer 32 thus forming a transition zone 38. By "integral with" is meant in intimate contact with but not requiring physical or chemical bonding. The structure depicted in Figure 3 is in an uncompressed state for ease of illustration. Upon compression, some of the portions in the wicking layer 34 will extend into and become integral with the fibers of the absorbing layer. These wicking layer portions consequently will also

be in contact with the superabsorbent material. Generally, at least 10 percent moisture is present when the structure is compressed under a pressure sufficient to compact the structure and cause the softened surface of the superabsorbent material to provide the necessary adhesion to the fibers of the absorbing layer so that the composite remains in a compacted state even when dry.

Figure 4 provides a cross-section view along line 4-4 of Figure 3 showing in detail the relationship of the layers of the starting material. The absorbing layer 42 is generally made from resilient staple fibers. The superabsorbent material 44 is interspersed and preferably fixed among the resilient fibers 43. The wicking layer 48 is comprised of portions 46, some of which extend into and become integral with the absorbing layer. The transition zone 45 contains the wicking layer portions 46 in contact with the portion of the absorbing layer 42 and its fibers 43 so as to be in intimate contact with some of the superabsorbent particles 44.

Figure 4A depicts the structure of Figure 4 in a compressed state showing that the absorbing layer 42A has been substantially reduced in thickness and the wicking layer 48A has also been reduced in thickness but extends considerably into and becomes integral with the absorbing layer to form a transition zone 45A. Although the superabsorbent particles 44A are closer to each other, there is still sufficient opportunity for liquid to pass between the particles and upon their softening the resilient fibers of the absorbing layer are released to return the layer to its original low density form. The compressed product depicted in Figure 4A is substantially stiff and may have a Taber stiffness of at least 300 in the machine direction.

Figure 5 represents a perspective view of yet another starting material which can be utilized to make the compressed composite product which is softened in the present invention. The starting material 50 is a fibrous web 52 containing at least 200 percent superabsorbent by weight of the web. The superabsorbent material 56 is distributed throughout the fibrous web 52. A wicking layer 54 is placed on each side of the fibrous web 52. The wicking layer generally is wood pulp fibers. When the product is compressed, a transition zone 58 is formed at the junction of each side of the absorbing layer, i.e. fibrous web 52, with the wicking layers 54.

Figure 6 denotes a section of the product of Figure 5 which has a fibrous web as an absorbing layer 62. Interspersed among the absorbing layer fibers 63 is superabsorbent material 64. Immediately associated with the absorbing layer is the wicking layer 68. Some portions of the wicking layer fibers 66 extend into and become integral with the absorbing layer 62, thus forming a transition zone 65. The structure depicted in Figure 6A is a compressed version of Figure 6. Upon compression, some of the portions in the wicking layer 68A will extend into and become integral with the fibers of the absorbing layer 62A. These wicking layer portions will also be in contact with the superabsorbent material 64A. Generally at least 10% moisture is present when the structure is compressed under a pressure sufficient to compact the structure and cause the softened surface of the superabsorbent material to provide the necessary adhesion to the fibers of the absorbing layer so that the composite remains in a compacted state even when dry.

Figure 7 is a perspective view of an absorbent structure 70 of the present invention comprising the starting material of Figure 6A which has been microcorrugated. The structure contains wicking layers 72, an absorbing layer 74, and microcorrugation hinge lines 76 and 78 which provide a Taber stiffness less than 25 in at least one direction.

Figure 8 depicts a disposable diaper 80 utilizing an absorbent structure of the present invention. A portion of the drawing is broken away for clarification. The disposable diaper 80 has a liquid-permeable facing 86 and a liquid-impermeable backing 82. In between the facing 86 and backing 82 is an absorbent structure 84. The structure has wicking layers, a transition zone, and an absorbing layer as described for the products of Figures 5 and 6 and has been microcorrugated as shown in Figure 7. The absorbent structure 84 is held in place by glue lines 88 so that it is sandwiched between the facing 86 and the backing 82. Tape tabs 89 are affixed to two corners of the diaper product in order to fasten the product about the waist of the wearer.

Figure 9 is a perspective view of a sanitary napkin 90. The napkin is comprised of a liquid-impermeable shell 94 which contains an absorbent structure 96 and is covered over the upper surface with a liquid-permeable facing 92. The absorbent structure 96 is made in accordance with the present invention and is similar to that of Figure 7.

Figure 10 is an enlarged side elevational view showing a portion of the absorbent structure of Figure 7 in more detail. The absorbent structure 100 is comprised of wicking layers 102 placed on each side of an absorbing layer 104. Generally, the superabsorbent material is polymerized from a monomer while the monomer is in contact with the fibers of the absorbing layer 104. Whenever a monomer is polymerized on a fibrous web such as the absorbing layer 104, it tends to polymerize in large globules or film-like pieces at the interstices of the web fibers. This adheres tee fiber-crossovers to each other resulting in a stiffness imparted to the fibrous web. When the stiff web is subjected to microcorrugating, the superabsorbent

polymer is reduced in size by fracturing to create a network of substantially parallel hinge lines no more than 6.35 mm (1/4 inch) apart. The principle is to break-up the superabsorbent material without materially damaging the original fiber matrix of the fibrous web. In other words, the web is rendered flexible and soft without a substantial loss of tensile strength.

Figure 11 depicts an enlarged side elevational view of a portion of suitable microcorrugating rolls. The rolls 112 and 114 possess flutes 116 and 118 which intermesh as the rolls rotate in opposite directions. The flutes 116 and 118 create staggered parallel cross directional lines in the absorbent structure creating hinge lines which impart flexibility without substantially damaging the integrity and functionality of the structure.

Figure 12 is a graph comparing the rate of absorbency of an absorbent structure which has been microcorrugated (MC) to an absorbent structure (control) which has not been treated. The graph clearly shows that an absorbent structure which has been microcorrugated absorbs liquid much more rapidly than the control.

Figure 13 is a perspective view of an absorbent composite structure which, after microcorrugating, has been perf-embossed in accordance with a preferred embodiment of the present invention. The composite 130 contains a wicking layer 135 and an absorbing layer 132. Following the microcorrugating and the perf-embossing, hinge lines 136 and apertures 139 are placed in the structure providing a pattern as shown. The Taber stiffness of the product is at least 75% less than that of the product prior to treatment and in most instances is less than 15.

Figure 14 depicts a disposable diaper 50 utilizing a microcorrugated and perf-embossed absorbent composite structure of the present invention. A portion of the drawing is broken away for clarification. The disposable diaper 140 has a liquid-permeable facing 146 and a liquid-impermeable backing 142. In between the facing 146 and the backing 142 is an absorbent composite structure 144. The diaper 140 is laminated into unitary form by glue lines 148. It is provided with tape tabs 149 for securing the diaper product 140 about the waist of the wearer.

Figure 15 is a perspective view of a sanitary napkin 150. The napkin is comprised of a liquid-impermeable shell 154 which contains an absorbent composite structure 156 and is covered over the upper surface with a liquid-permeable facing 152. The absorbent structure 156 is made in accordance with the present invention and is similar to that of Figure 13.

Figure 16 is a graph depicting the test results of a test performed on the control sample and a sample prepared in accordance with the present invention. The test is derived from the GAT apparatus described and set forth in U.S. Patent 4,357,827 issued November 9th, 1982. The values given shown the absorbency of a simulated urine solution under a load of 3.4 kPa (0.5 psi) in different periods of time. The absorbency value is what the structure can absorb in the given time while the structure is under a load of 3.4 kPa (0.5 psi). The values given in Figure 16 will be discussed in Example 3.

The products depicted in the drawings and other products such as incontinence pads, wound dressings and the like may be made from the absorbent composite structure of the present invention.

Examples of methods of preparing the absorbent structure of the present invention are as follows.

Example 1

An absorbing layer is formed of polyester fibers by dry-laying the fibers, i.e. by air-laying or carding the fibers, to form a web. Specifically, the polyester fibers contain a minor proportion (about 15 percent) of fusible fibers which soften at a lower temperature than the rest of the fibers. The web is heat bonded by passing air at a temperature of 177° C (350° F) through the web for about 10 seconds. The resulting web is about 25 $g/m^2$ basis weight. The specific polyester fibers used are identified as Type 99 Hollofil fibers manufactured and sold by E.I. DuPont Company.

The polyester web is coated by flooding it with an aqueous solution of sodium acrylate and acrylic acid. The solution contains 38 percent solids. Excess solution is removed from the web and the web is then subjected to electron beam radiation in order to polymerize the sodium acrylate to polysodium acrylate. The web is repeatedly flooded with liquid, the excess liquid removed and subjected to irradiation until the amount of dry solids add-on of the polysodium acrylate is 10 times the weight of the web.

The coated substrate is passed beneath a hammer mill that deposits wood pulp fibers on to the polyester web. Vacuum is applied under the polyester web so as to lightly compact the wood pulp fibers on to the web. The wood pulp fibers are present in an amount of 50 $g/m^2$. The surface of the pulp layer is sprayed with water so that the total moisture content of the pulp is about 10 percent by weight. The total structure is compressed at a level of 4.4 MPa (640 psi) for 30 seconds. Upon release of the pressure, the pulp has formed into a high density layer with a capillary size suitable for liquid wicking and the resilient fiber layer remains compressed.

The sample when dried to 6 percent moisture has a Taber stiffness of 158. This stiffness does not permit use of the absorbent structure in products such as disposable diapers and sanitary napkins. Addition of moisture up to about 28 percent softens the product to a Taber stiffness of about 75. However, it still is not sufficiently soft and flexible to be used in a disposable diaper product. Furthermore, it is desirable for the absorbent structure to be substantially dry when used in a product so that it can absorb its full capacity of liquid when being used. Upon redrying the product after bringing the moisture to 28 percent, the product at 3 percent moisture has a Taber stiffness value of 119 and at 0 percent moisture has a Taber stiffness value of 145.

Another sample is dried to 6 percent moisture and subjected to microcorrugating rolls first through a pair of intermeshing rolls with a corrugating pattern in the cross-direction followed by passing the sample through an embossing roll with rings that create lines in the machine direction. The resulting Taber stiffness value is about 21 in the machine direction. When this sample is moisturized to about 28 percent, the Taber stiffness value drops to about 13. When the sample is redried to 3 percent and 0 percent moisture, the Taber stiffness values are about 21 and 22, respectively. This clearly shows that by drying the sample before microcorrugating, the microcorrugating is permanent and the sample will remain soft and flexible whether or not it is moisturized and subsequently dried. When a sample is microcorrugated a second time in each direction, the Taber stiffness values are as follows: at 6 percent moisture - 11, at 28 percent moisture - 10, redried to 3 percent moisture - 21, redried to 0 percent moisture - 23. A Taber stiffness value below 50 produces a satisfactory product but the preferred value is less than 25.

Example 2

A polyester nonwoven fibrous web is prepared which contains 67 percent Hollofil fibers and 33 percent Enka fibers. The Enka fibers are a bicomponent fiber having a polyethylene sheath and a polyester core. These fibers are produced and sold by American Enka. The fabric has a weight of 40.7 g/m$^2$ (1.2 oz/yd$^2$). In a similar procedure to that in Example 1, the polysodium acrylate is placed on the fibers in an amount of dry solids add-on of 10 to 1. The final weight of the superabsorbent containing polyester web is 406.9 g/m$^2$ (12 oz/yd$^2$). After formation of the web, approximately 135.6 g/m$^2$ (4 oz/yd$^2$) on each side of the web of wood pulp fibers are deposited and the web is compressed. When dried to a moisture level of 3 percent, the web exhibits a Taber stiffness of 343 cm in the machine direction and 75.8 cm in the cross-direction. Samples of the web (25.4 x 38.1 cm) (10 inches x 15 inches), are dried at 190 °C (375 °F) to a moisture content of 3 percent. The samples are then processed through the microcorrugating rolls in the machine direction. The rolls are 17.8 cm (7 inch) cylinders having microcorrugated peaks at 76.2 cm (30 inch) dp (diametral pitch) with an engagement of 1.02 mm (0.04 inch) at a pressure of 50.8 N/mm (290 pounds per lineal inch). The samples are then placed through the embossing rolls with rings to microcorrugate the product in the cross-direction. These samples after microcorrugation have a Taber stiffness value of 26.5 in the machine direction and 15 in the cross-direction. The microcorrugated samples reveal a higher absorption of liquid and a more rapid absorption of liquid as shown in Figure 12. The wicking ability of the microcorrugated product is substantially equal to that of the product prior to microcorrugating. Thus, the mechanical working by microcorrugation has not interfered with the ability of the product to wick liquid from one region of the product to another. This is very important in a structure such as a disposable diaper.

Example 3

An absorbing layer for a compressed composite is formed of 67 percent polyester fibers and 33 percent bicomponent fibers. The bicomponent fibers have a polyethylene sheath and a polyester core. The web is heat bonded by passing air at a temperature of 177 °C (350 °F) through the web for one second or less. The resulting web has a weight of 40.7 g/m$^2$ (1.2 oz/yd$^2$). The web is coated by flooding it with an aqueous solution of sodium acrylate and acrylic acid. The solution contains 38 percent solids. Excess solution is removed from the web and the web is then subjected to electron beam radiation. This electron beam radiation polymerizes the sodium acrylate to polysodium acrylate. The web is repeatedly flooded with liquid, the excess liquid removed, and each time subjected to irradiation until the amount of dry solids add-on of the polysodium acrylate is 10 times the weight of the web.

The polysodium acrylate coated web is passed beneath a hammer mill that deposits wood pulp fibers onto the polyester web. Vacuum is applied under the polyester web so as to lightly compact the wood pulp fibers onto the web. The wood pulp fibers are present in an amount of 135.6 g/m$^2$ (4 oz/yd$^2$) per layer and a layer of the wood pulp fibers is deposited on each side of the polyester web. The surface of the pulp layer is sprayed with water so that the total moisture content of the pulp is at least 10 percent by weight. The

total structure is then compressed at a level of 4.4 MPa (640 psi) for 30 seconds. Upon release of the pressure, the pulp has formed into a higher density layer with a capillary size suitable for liquid wicking and the resilient fiber layer remains compressed. The product containing about 20 percent moisture has a Taber stiffness in the machine direction of 343, and in the cross-direction of 75. If the compressed composite is subjected to perf-embossing at 20 percent moisture, the Taber stiffness in the machine direction increases to about 376 while there is a slight reduction in cross-direction Taber stiffness to a value of about 65. In all instances, the Taber stiffness values are presented in grams per lineal centimeter of the sample.

The compressed composite product containing about 20% moisture is dried to a moisture content of about 3% and subjected to microcorrugating in the machine direction. The rolls are 17.8 cm (7 inch) cylinders having microcorrugated peaks at 76.2 cm (30 inch) dp (diametral pitch) with an engagement of 1.02 mm (0.04 inch) at a pressure of 50.8 N/mm (290 lbs per lineal inch). These samples, after microcorrugation have a Taber stiffness value of about 27 in the machine direction and 15 in the cross-direction. The sample is then subjected to perf-embossing wherein the rolls are set at 1.27 mm (0.05 inch) engagement with 276 KPa (40 psi). The Taber stiffness of the product which has been microcorrugated and perf-embossed in the machine direction is reduced to 8.0 and in the cross direction to 3.6. It can be clearly seen that the stiffness in the product is reduced by at least 75% in each direction. The mechanically worked product exhibits an improved absorbency in that it shows a 32% increase in absorbency after being mechanically worked.

The results of an absorbency test in given time periods are shown in Figure 16 in graph form. It is readily seen that the sample treated by microcorrugating and perf-embossing (MC/PE) much more rapidly absorbs liquid in a given time period than an untreated compressed composite product.

From the foregoing it will be observed that numerous variations and modifications may be effected without departing from the scope of this invention as defined in the following claims.

## Claims

1. An absorbent structure comprising a fibrous web of resilient fibers, said web having a basis weight less than 135.6 g/m$^2$ (4oz/yd$^2$), an initial dry bulk of at least 20 cm$^3$/g, a dry bulk recovery of at least 30 percent and a wet bulk of at least 30 cm$^3$/g, and superabsorbent disposed in amongst the fibers of said web in an amount of at least 200 percent by weight based on said web weight, said structure having a Taber stiffness value of less than 50 and being microcorrugated.

2. The structure of claim 1, wherein the Taber stiffness value is less than 25.

3. The structure of claim 1 or claim 2, further comprising a wicking layer placed on at least one face of said fibrous web.

4. The structure of claim 3, wherein there is a wicking layer placed on each face of said fibrous web.

5. The structure of claim 3 or claim 4, wherein the or each wicking layer comprises wood pulp fibers.

6. The structure of any one of claims 3 to 5, wherein the Taber stiffness value is less than 10.

7. The structure of any one of claims 1 to 6, wherein the superabsorbent is present in an amount from 200 to 1500 percent by weight.

8. The structure of claim 7, wherein the superabsorbent is present in an amount from 400 to 1200 percent by weight.

9. The structure of any one of claims 1 to 8, wherein said fibrous web is a nonwoven web of polyester fibers.

10. A disposable diaper or a sanitary napkin comprising, as an absorbent layer, a structure of any one of claims 1 to 9.

11. A method for preparing a soft, flexible absorbent structure comprising the steps of:
    a) preparing a fibrous web of resilient fibers, said web having a basis weight less than 135.6 g/m$^2$ (4 oz/yd$^2$), an initial dry bulk of at least 20 cm$^3$/g, a dry bulk recovery of at least 30 percent and a wet

bulk of at least 30 $cm^3/g$, said web having superabsorbent disposed in amongst its fibers in an amount of at least 200 percent by weight based on said web weight;
b) drying said web containing superabsorbent to a moisture content less than 25 percent; and
c) microcorrugating said web to provide a Taber stiffness of less than 50.

**12.** The method of claim 11, wherein said Taber stiffness value is reduced to less than 25.

**13.** The method of claim 12, wherein said Taber stiffness value is less than 10 in at least one direction.

**14.** The method of any one of claims 11 to 13, wherein the moisture content is reduced to less than 10% prior to microcorrugating.

**15.** The method of any one of claims 11 to 14, wherein before or after said microcorrugating step (c) a wicking layer is placed on at least one face of said fibrous web and said structure including said wicking layer is subjected to perf-embossing following the microcorrugating step.

**16.** The method of claim 15, wherein a wicking layer is placed on each face of the fibrous web.

**Revendications**

**1.** Structure absorbante comprenant une nappe fibreuse en fibres flexibles, ladite nappe ayant un grammage inférieur à 135,6 $g/m^2$ (4 $oz/yd^2$), un volume initial sec d'au moins 20 $cm^3/g$, un rappel du volume sec d'au moins 30% et un volume à l'état humide d'au moins 30 $cm^3/g$, et un superabsorbant interposé entre les fibres de ladite nappe en une quantité d'au moins 200% par rapport au poids de ladite nappe, ladite structure présentant une rigidité Taber de moins de 50 et étant micro-ondulée.

**2.** Structure selon la revendication 1, dans laquelle la rigidité Taber est inférieure à 25.

**3.** Structure selon la revendication 1 ou 2, qui comprend en outre une couche à effet de mèche placée sur au moins une face de ladite nappe fibreuse.

**4.** Structure selon la revendication 3, dans laquelle une couche à effet de mèche est placée sur chaque face de ladite nappe fibreuse.

**5.** Structure selon la revendication 3 ou 4, dans laquelle la ou chaque couche à effet de mèche comprend des fibres de pâte de bois.

**6.** Structure selon l'une quelconque des revendications 3 à 5, dans laquelle la rigidité Taber est inférieure à 10.

**7.** Structure selon l'une quelconque des revendications 1 à 6, dans laquelle le superabsorbant est présent à raison de 200 à 1500% en poids.

**8.** Structure selon la revendication 7, dans laquelle le superabsorbant est présent à raison de 400 à 1200% en poids.

**9.** Structure selon l'une quelconque des revendications 1 à 8, dans laquelle ladite nappe fibreuse est une nappe non tissée en fibres de polyester.

**10.** Couche ou serviette hygiénique jetable comprenan, à titre de couche absorbante, une structure selon l'une quelconque des revendications 1 à 9.

**11.** Procédé de préparation d'une structure absorbante douce et flexible, qui consiste :
a) à préparer une nappe fibreuse en fibres flexibles, ladite nappe ayant un grammage inférieur à 135,6 $g/m^2$ (4 $oz/yd^2$), un volume sec initial d'au moins 20 $cm^3/g$, un rappel de volume à sec d'au moins 30% et un volume à l'état humide d'au moins 30 $cm^3/g$, ladite nappe comportant un superabsorbant interposé entre ses fibres en une quantité d'au moins 200% par rapport au poids de ladite nappe ;

b) à sécher ladite nappe contenant un superabsorbant jusqu'à une teneur en humidité inférieure à 25% ; et
c) à micro-onduler ladite nappe pour obtenir une rigidité Taber inférieure à 50.

**12.** Procédé selon la revendication 11, dans lequel ladite rigidité Taber est réduite à moins de 25.

**13.** Procédé selon la revendication 12, dans lequel la rigidité Taber est inférieure à 10 dans au moins une direction.

**14.** Procédé selon l'une quelconque des revendications 11 à 13, dans lequel on réduit la teneur en humidité à moins de 10% avant la micro-ondulation.

**15.** Procédé selon l'une quelconque des revendications 11 à 14, dans lequel avant ou après ledit stade de micro-ondulation (c), on place une couche à effet de mèche sur au moins une face de ladite nappe fibreuse et on soumet ladite structure comportant ladite couche à effet de mèche à un perfo-gaufrage faisant suite au stade de micro-ondulation.

**16.** Procédé selon la revendication 15, dans lequel on place une couche à effet de mèche sur chaque face de la nappe fibreuse.

**Patentansprüche**

**1.** Absorbierende Struktur, welche eine Faserbahn aus elastischen Fasern umfaßt, welche Faserbahn ein Flächengewicht von weniger als 135,6 g/m$^2$ (4 Unzen je Quadratyard), ein anfängliches spezifisches Trockenvolumen von wenigstens 20 cm$^3$/g, ein Rückstellvermögen des spezifischen Trockenvolumens von wenigstens 30% und ein spezifisches Naßvolumen von wenigstens 30 cm$^3$/g aufweist, und welche Faserbahn ein zwischen den Fasern dieser Bahn in einer Menge von wenigstens 200 Gew.%, bezogen auf das Gewicht der Faserbahn, angeordnetes, superabsorbierendes Material aufweist, wobei diese Struktur einen Taber-Steifheitswert von weniger als etwa 50 aufweist und mikrogeriffelt ist.

**2.** Struktur nach Anspruch 1, wobei der Taber-Steifheitswert weniger als 25 beträgt.

**3.** Struktur nach Anspruch 1 oder 2, welche weiterhin eine auf wenigstens einer Seite der Fasernbahn angeordnete, dochtartig aufsaugende Schicht umfaßt.

**4.** Struktur nach Anspruch 3, wobei auf jeder Seite der Faserbahn eine dochtartig aufsaugende Schicht angeordnet ist.

**5.** Struktur nach Anspruch 3 oder 4, worin die dochtartig aufsaugende Schicht oder jede dochtartig aufsaugende Schicht Holzzellstoffasern enthält.

**6.** Struktur nach einem der Ansprüche 3 bis 5, worin der Taber-Steifheitswert weniger als 10 beträgt.

**7.** Struktur nach einer der Ansprüche 1 bis 6, worin das superabsorbierende Material in einer Menge von 200 Gew.-% bis 1500 Gew.-% vorliegt.

**8.** Struktur nach Anspruch 7, worin das superabsorbierende Material in einer Menge von 400 Gew.-% bis 1200 Gew.-% vorliegt.

**9.** Struktur nach einem der Ansprüche 1 bis 8, worin die Faserbahn eine Faservliesstoffbahn aus Polyesterfasern ist.

**10.** Wegwerfwindel oder Damenbinde, welche als absorbierende Schicht eine Struktur nach einem der Ansprüche 1 bis 9 umfaßt.

**11.** Verfahren zur Herstellung einer weichen, biegsamen, absorbierenden Struktur, welches Verfahren die folgenden Stufen umfaßt:
a) Herstellen einer Faserbahn aus elastischen Fasern, welche Faserbahn ein Flächengewicht von

weniger als 135,6 g/m$^2$, (4 Unzen je Quadratyard), ein anfängliches spezifisches Trockenvolumen von wenigstens 20 cm$^3$/g, ein Rückstellvermögen des spezifischen Trockenvolumens von wenigstens 30 % und ein spezifisches Naßvolumen von wenigstens 30 cm$^3$/g aufweist, und welche Faserbahn ein zwischen den Fasern dieser Bahn in einer Menge von wenigstens 200 Gew.-%, bezogen auf das Gewicht der Faserbahn, angeordnetes, superabsorbierendes Material aufweist;
b) Trocknen der das superabsorbierende Material enthaltenden Bahn auf einen Feuchtigkeitsgehalt von weniger als 25%; und
c) Mikroriffeln dieser Bahn, um einen Taber-Steifheitswert von weniger als 50 zu erzielen.

**12.** Verfahren nach Anspruch 11, wobei der Taber-Steifheitswert auf weniger als 25 verringert wird.

**13.** Verfahren nach Anspruch 12, wobei der Taber-Steifheitswert in wenigstens einer Richtung weniger als 10 beträgt.

**14.** Verfahren nach einer der Ansprüche 11 bis 13, wobei der Feuchtigkeitsgehalt vor dem Mikroriffeln auf weniger als 10 % verringert wird.

**15.** Verfahren nach einem der Ansprüche 11 bis 14, wobei vor oder nach der Stufe (c), dem Mikroriffeln, auf wenigstens einer Seite der Faserbahn eine dochtartig aufsaugende Schicht angeordnet wird, und diese Struktur, einschließlich der dochtartig aufsaugenden Schicht, im Anschluß an die Stufe des Mikroriffelns einer Perforierung und Prägung unterworfen wird.

**16.** Verfahren nach Anspruch 15, wobei auf jeder Seite der Faserbahn eine dochtartig aufsaugende Schicht angeordnet wird.

FIG-1

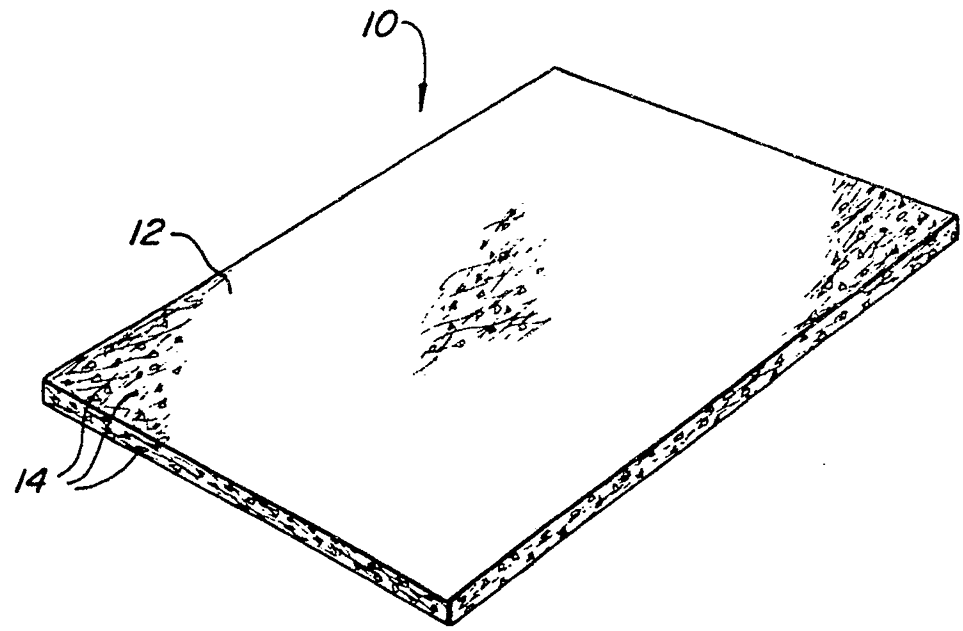


FIG-2

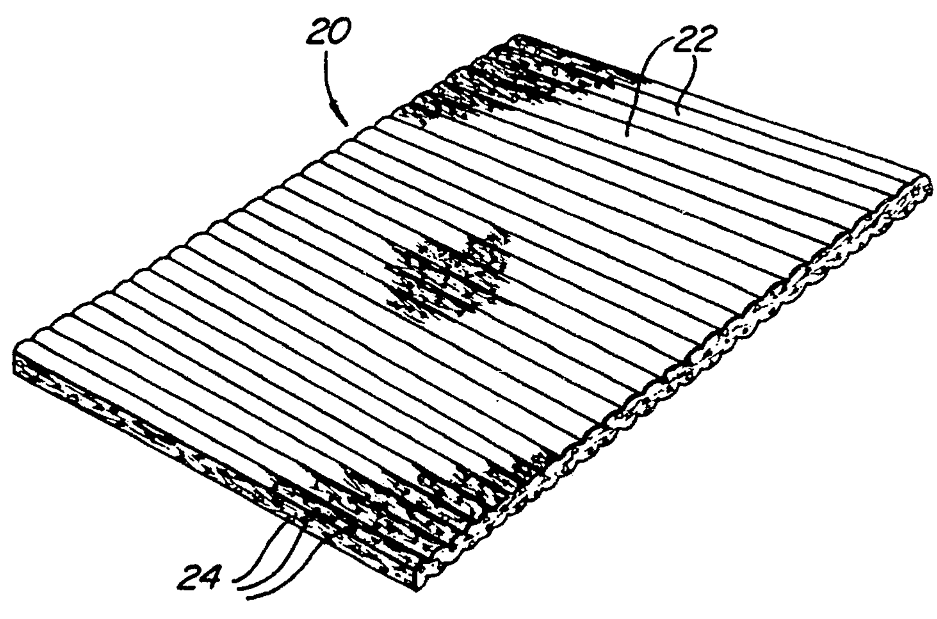

FIG-3

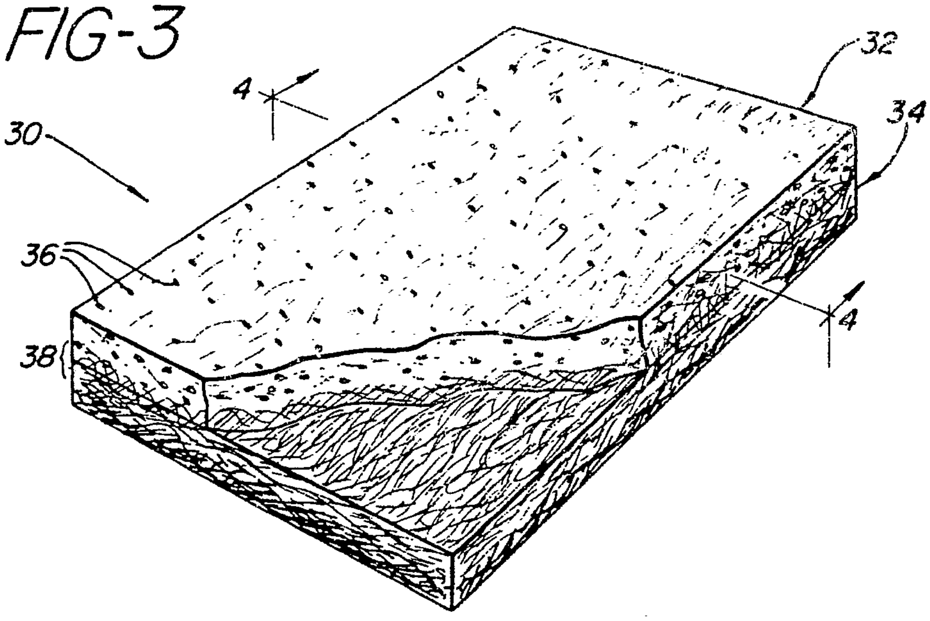


FIG-4

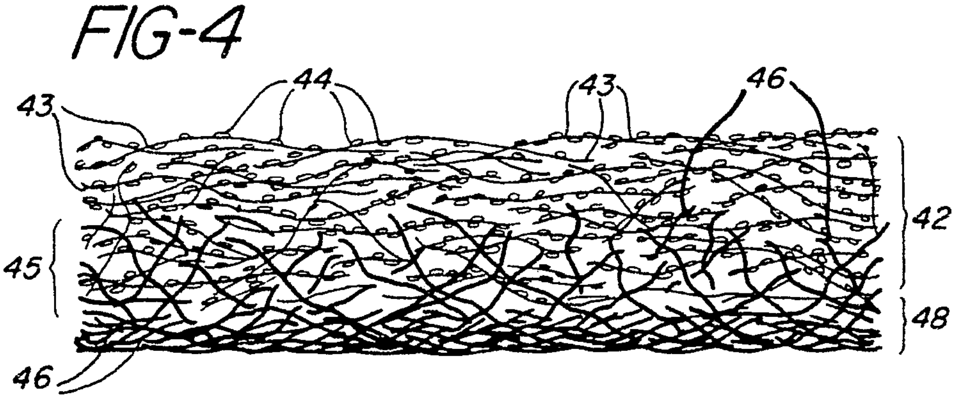


FIG-4A

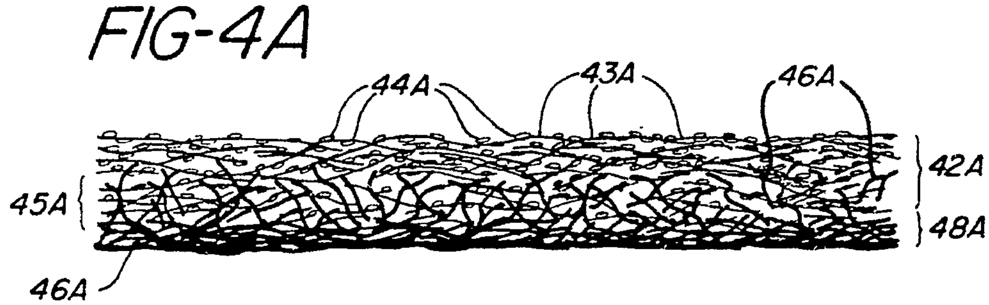

FIG-5

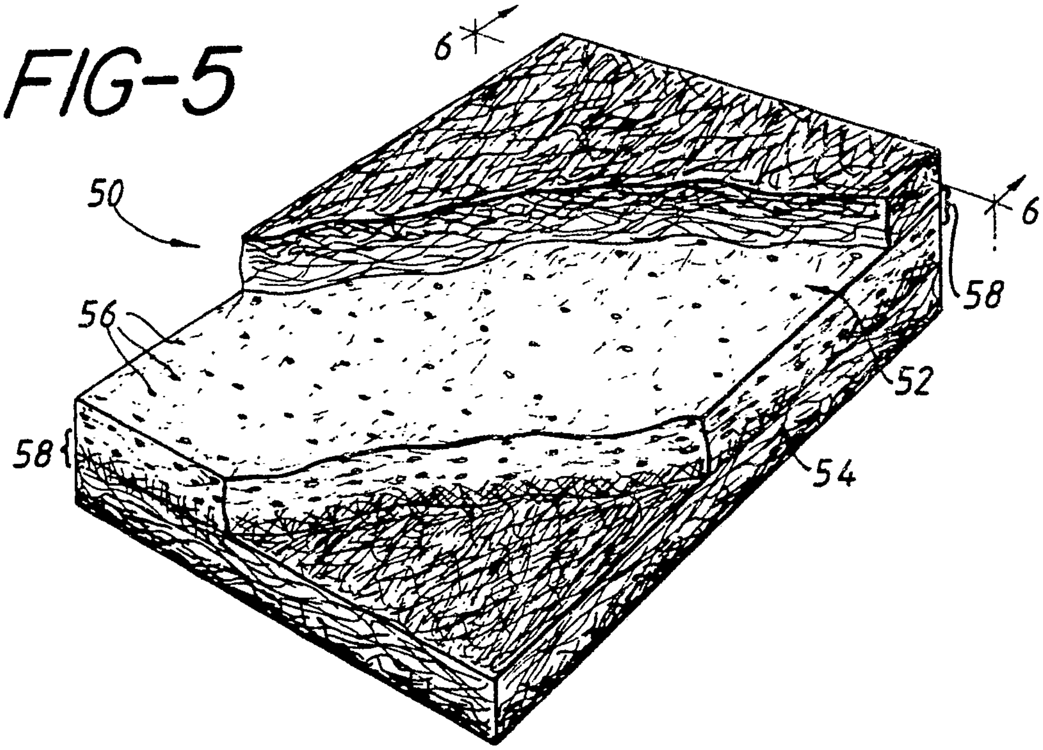


FIG-6

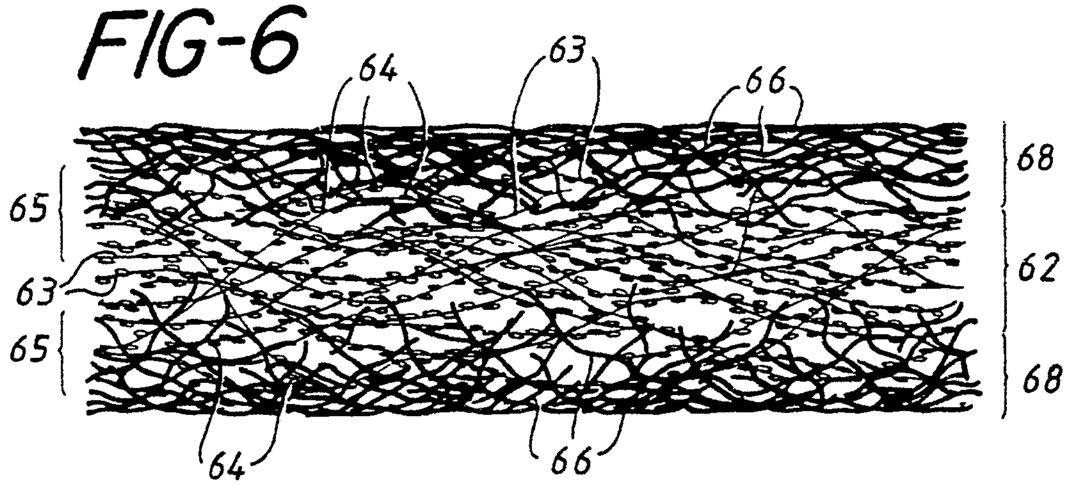


FIG-6A

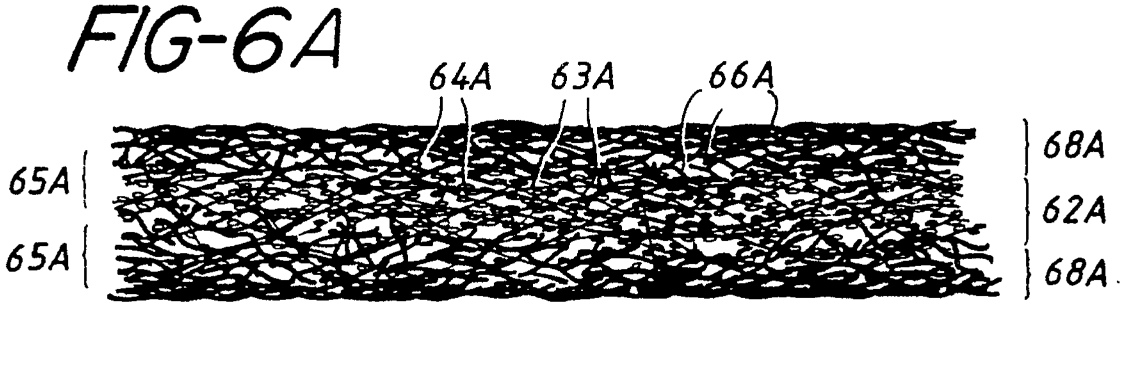

FIG-7

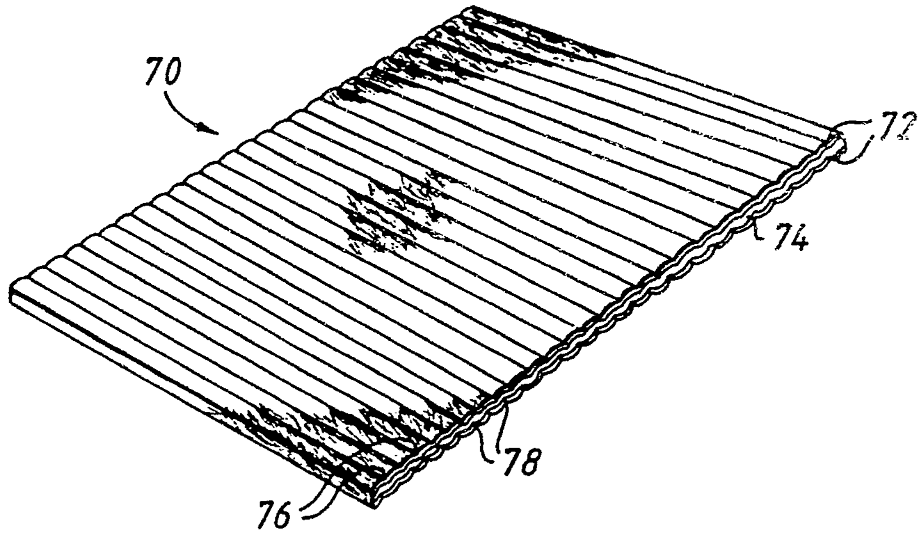


FIG-8

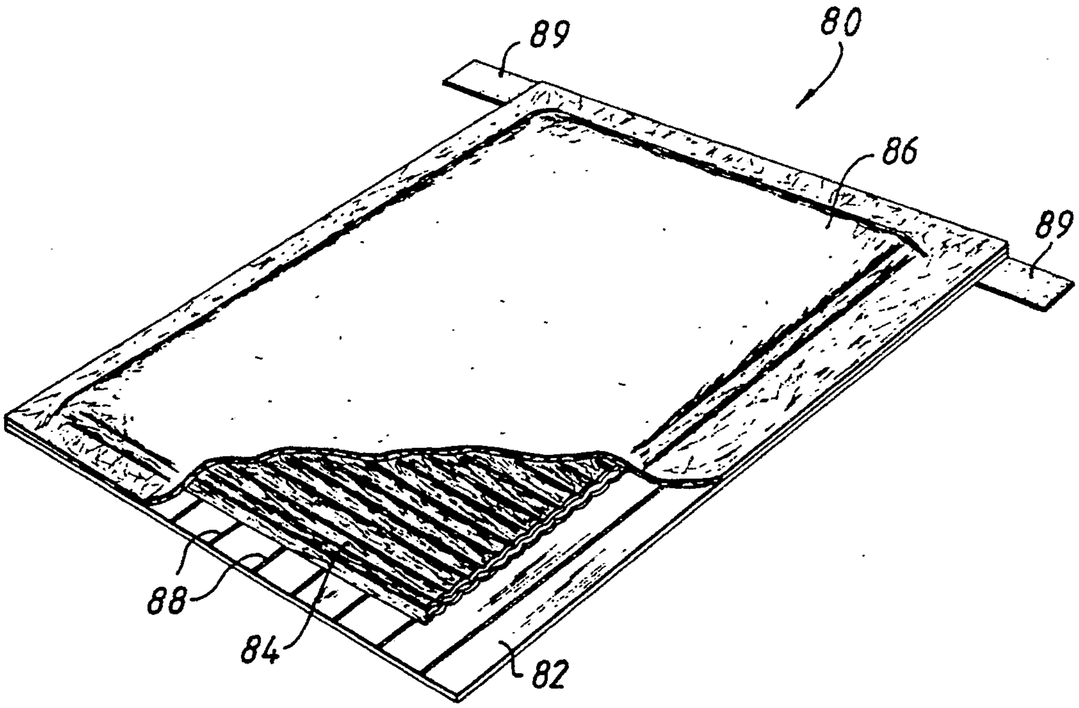

FIG-9

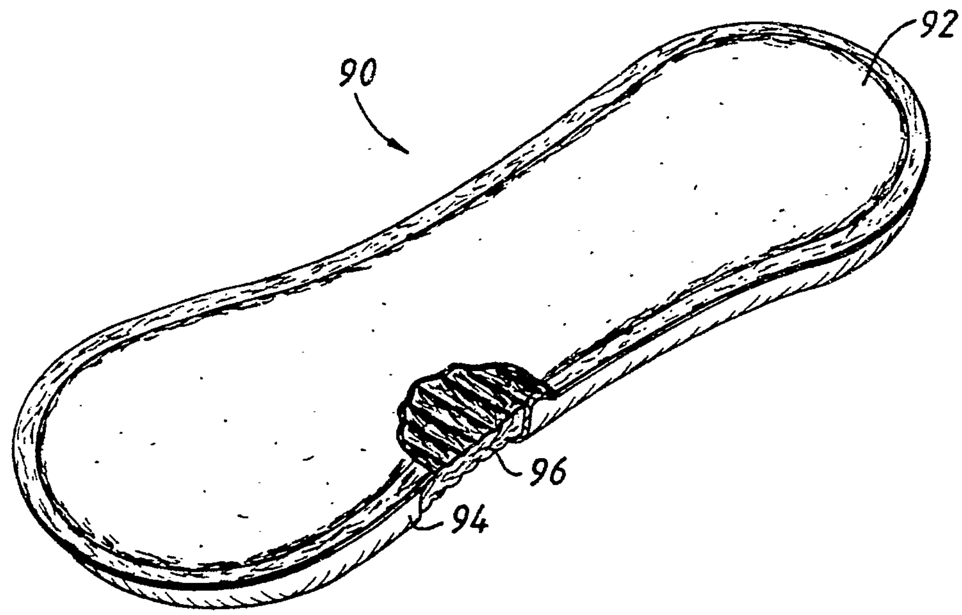


FIG-10

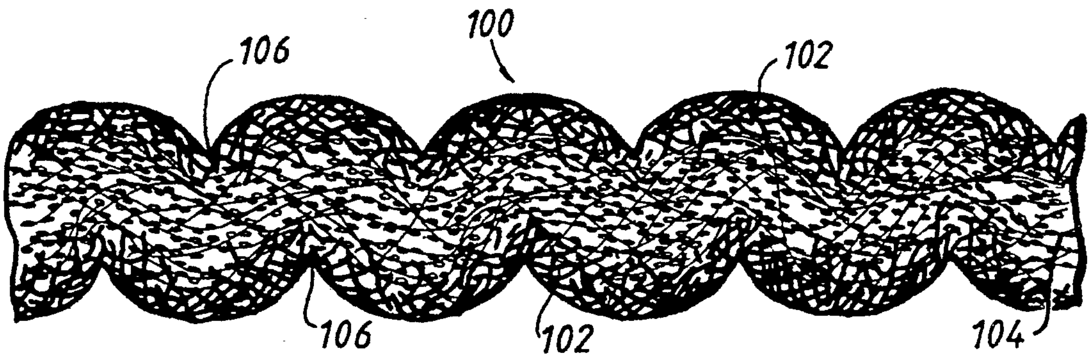


FIG-11

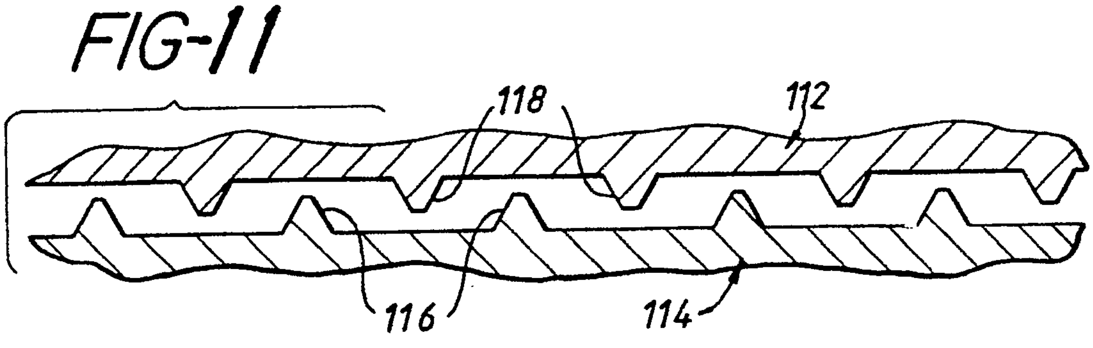

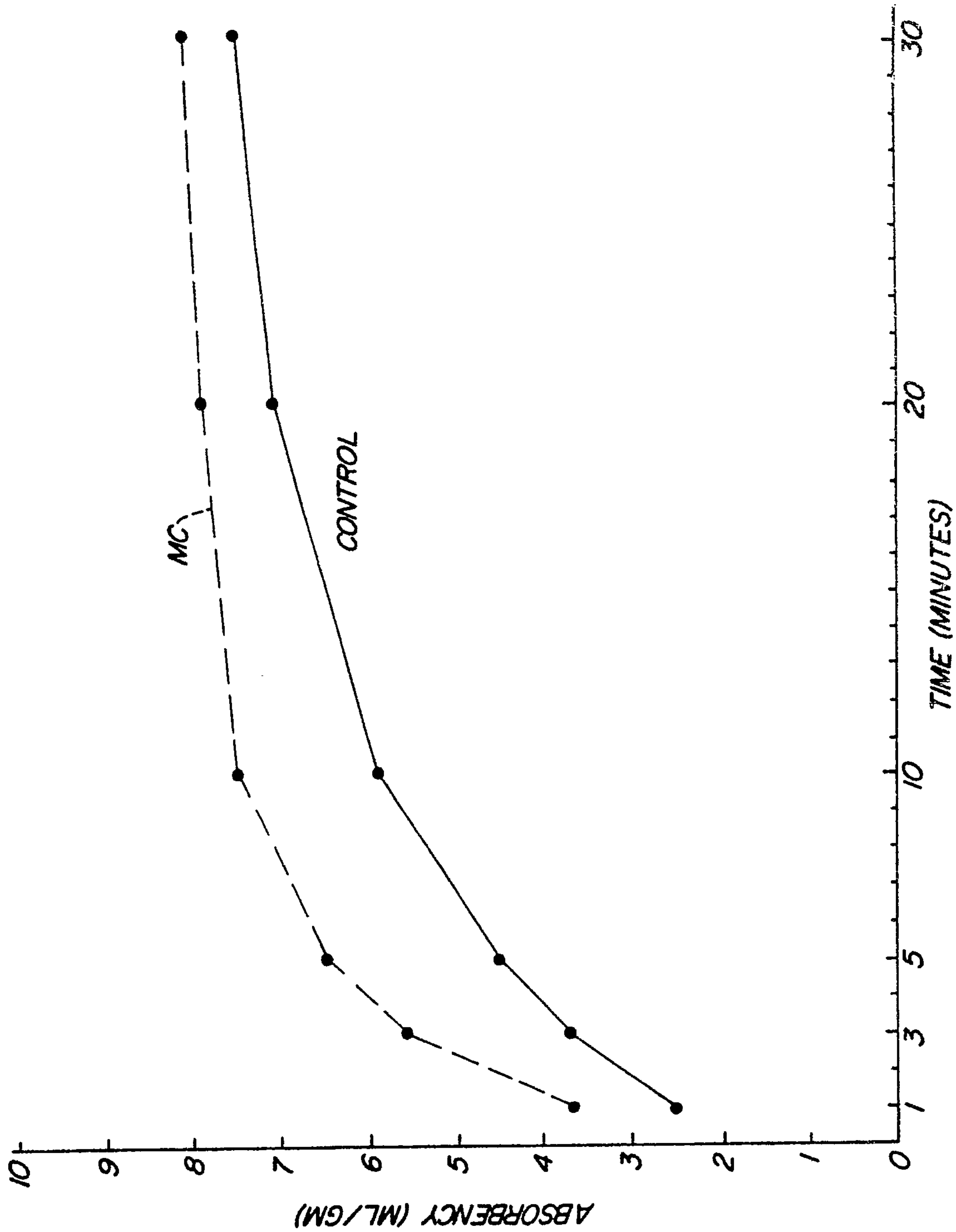
ABSORBENCY (ML/GM)
0
1
2
3
4
5
6
7
8
9
10
TIME (MINUTES)
1
3
5
10
20
30
MC
CONTROL

FIG-12

FIG-13

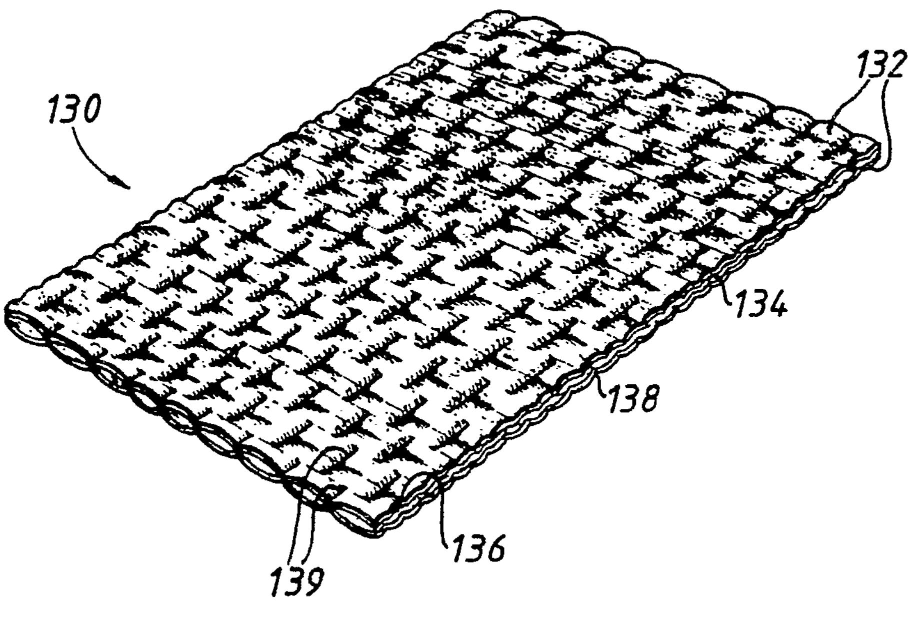

FIG-14

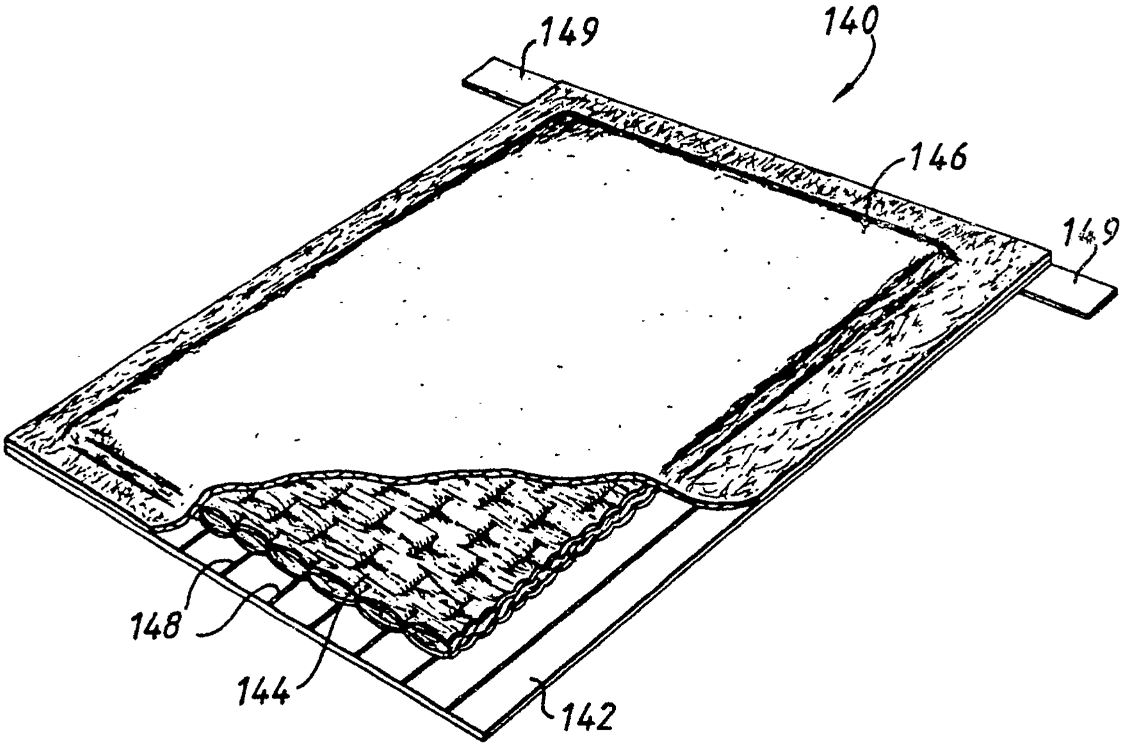


FIG-15

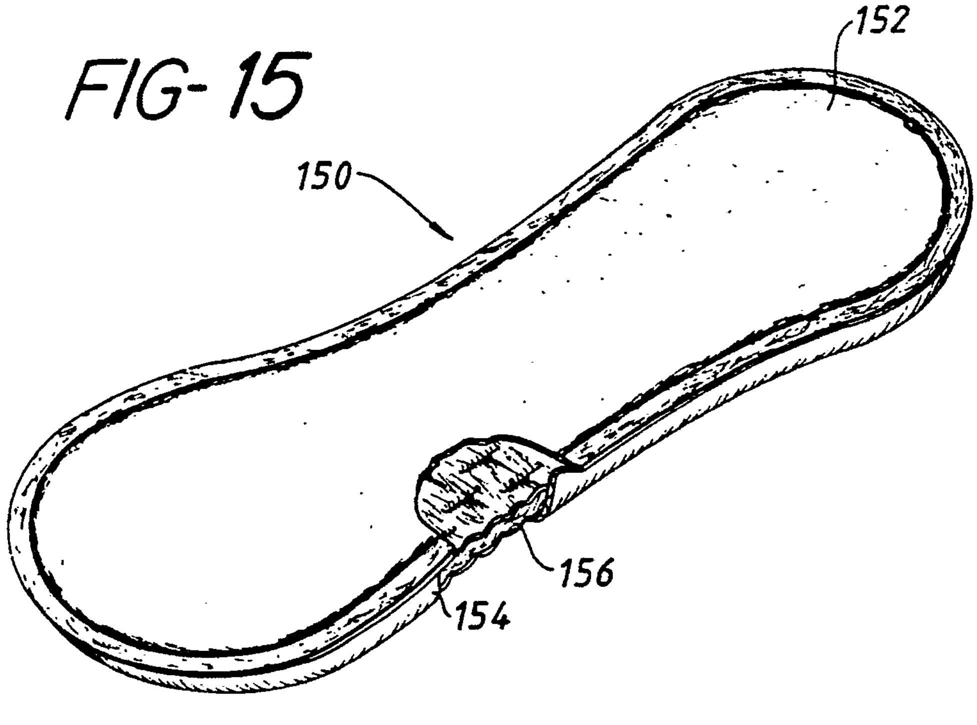

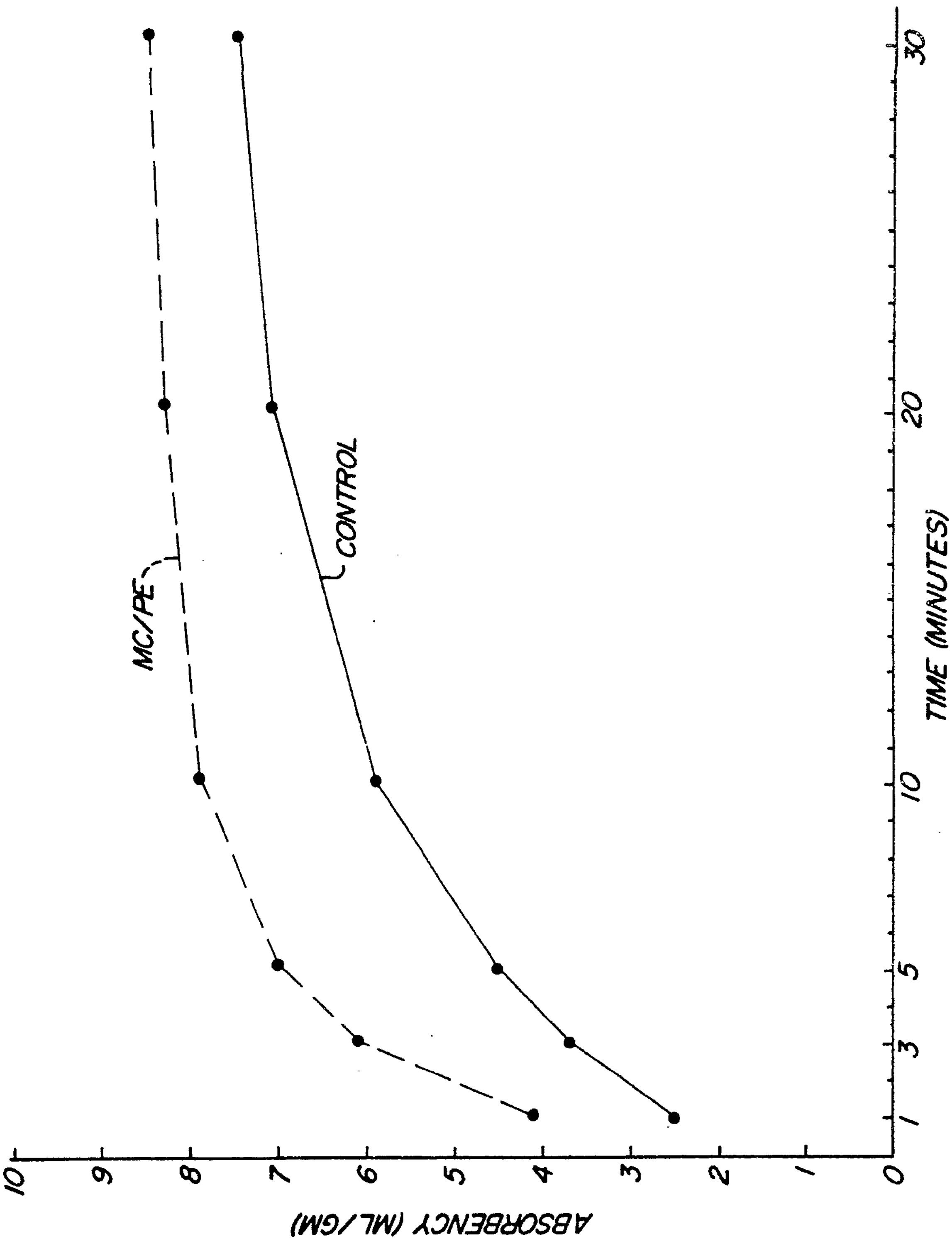
ABSORBENCY (ML/GM)
TIME (MINUTES)
MC/PE
CONTROL
0
1
2
3
4
5
6
7
8
9
10
1
3
5
10
20
30

FIG-16